# EUROPEAN PATENT APPLICATION

(11) **EP 2 722 802 A2**
(43) Date of publication of application: **23.04.2014**
(21) Application number: 13189170.7
(22) Date of filing: 17.10.2013
(51) Int. Cl.: G06Q 20/36

(54) **Multi-function device**

(30) Priority: 19.10.2012 IT BO20120104 U
(71) Applicant: Ideas4Life di Buriani Luca, 44011 Argenta (Ferrara) (IT)
(72) Inventor: Buriani, Luca, 44011 ARGENTA (FERRARA) (IT)
(74) Representative: Dall'Olio, Christian

(57) **Abstract**

Multi-function device (1) associated to a user, comprising at least two of the following features: at least a part of data related to a driving license (2); at least a part of data related to a health insurance card (3, 9); at least a part of data related to an identity card (4); means of authorization/access to telematic payment services (5); means for storing data (8).

## Description

The present invention relates to a multi-function device, in particular of a type associated to an user.

It is known that, in recurring or daily circumstances, it is necessary to produce documents that allow one's own identification.

Consider, for example, reservations of various kinds (hotels, airline tickets...), as well as access to services (banking services, postal services, health services..), or stipulation of contracts of various kinds (subscription to telephone company, etc.). In these cases, it is necessary to have one's own identity card and/or health insurance card, in which is present the tax code of the user.

In addition, it is often necessary to have one's own driving license, to avoid incurring penalties in case of inspections by the police, or for example in case of a rental vehicle.

Therefore, it is necessary to have bulky wallets, bags or shoulder bags in order to always have at one's disposal all instruments and documents mentioned above, that, otherwise, are liable to be forgotten or lost.

Furthermore, in some situations it happens it is necessary to have various kinds of paper documents, such as medical certificates, reports, prescriptions, invoices/receipts of payment, etc.. The material indicated above is generally placed inside folders or envelopes of different sizes, folders or envelopes that are not very practical and inside which documents are liable to be damaged.

The purpose of the present invention is to overcome the aforementioned problems providing a multi-function device according to claim 1.

Advantageously, the device proposed with this invention allows a user to have at his disposal, with a single instrument, a plurality of documents and/or to access to many services.

Therefore, the user does not necessarily have to equip himself with bulky wallets, bags or shoulder bags of various type such as in prior art in order to use a document or a service.

In prior art, further, numerousness of documents/instruments simultaneously necessary in some situations could lead the user to forget to bring with him one or more of these documents /instruments, causing considerable problems.

Additionally, the proposed device allows to have at one's disposal documents, certificates of any kind, invoices/receipts of payment that in prior art were carried into folders or bulky envelopes.

The device according to the present invention prevents prior art drawbacks and it is also practical to be carried.

Specific embodiments of the invention and additional and advantageous features will be made evident in the following discussion, with the aid of the enclosed drawing, in which:
- Figure 1 shows a side view of a multi-function device proposed with the present invention, in which are represented symbols and alphanumeric characters by way of example;
- Figure 2 shows a different side view of the device of figure 1, in which are represented alphanumeric characters and symbols by way of example.

With reference to the attached figures, it has been indicated with 1 a multi-function device according to the present invention.

The multi-function device 1 is of a type associated to a user and comprises at least two of the following features: at least a part of data related to a driving license 2; at least a part of data related to a health insurance card 3, 9; at least a part of data related to an identity card 4; means of authorization/access to telematic payment services 5; means for storing data 8.

In particular, in the preferred embodiment illustrated in the figures, the device 1 includes all the features mentioned above.

Obviously, in other embodiments, not illustrated, the device 1 can include any combination of the features mentioned above.

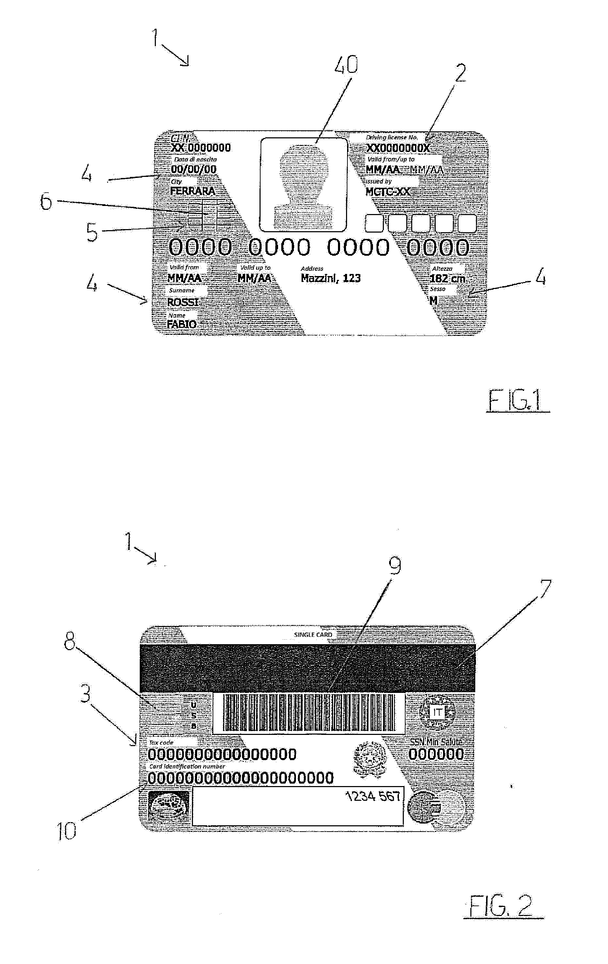

In particular, with reference to the attached figures, the data related to a driving license 2 include the driving license number (or, eventually, the driving licenses numbers, if more than one) of the user, the period of validity of the driving license and the name of institution that issued it (see Figure 1).

The data related to a health insurance card can include the tax code 3 of the user and/or a bar code 9 for access to health services (see Figure 2).

The bar code 9 is univocally bound to the user, and apart from being useful for access to health services/health care (as in the case of the bar code present on "conventional" health insurance cards), can be employed for the validation of tickets for trains, buses, subways, as well as points collections of any kinds, etc..

The data related to an identity card 4, instead, can include the user's name and surname, the city/date of birth, the residence address, the height and the gender of the user (see Figure 1, in which the data are exclusively provided by way of example), as well as his passport photo 40 (represented in a schematic way).

Advantageously, the proposed device 1 may be a single document as replacement of the plurality of instruments/documents required in prior art, such as identity card, driving license and health insurance card. Therefore, the device 1 according to the invention prevents to have a plurality of documents/instruments and to have bulky wallets/bags/shoulder bags in order to contain all documents/instruments, and therefore it constitutes "one and only card".

Moreover, the device 1 allows the storage of data such as paper documents of any type: advantageously, the means for storing data 8, in fact, allows storing, and then having with oneself, documents such as certificates/medical reports, medical records, receipts/invoices of payment, certificates or forms of various kind.

Reading and storing data/documents of a user can be realized by a suitable reader, not shown in the attached drawings.

For example, the means for storing data comprise a USB memory 8 (see Figure 2).

Otherwise, reading and storing data/documents of a user can be realized with NFC (Near Field Communication) technology (situation not illustrated). In other words, said means for storing may include a NFC device.

Preferably, the device 1 is a card/tag and is therefore particularly practical to be carried.

The means of authorization/access to telematic payment services 5 are linked to ATM circuits and/or credit cards circuits.

In particular, said means for authorization/access to telematic payment services 5 include a security chip 6 and/or a magnetic band 7.

The chip 6 is of a type already present in the existing credit cards/debit cards, and in the illustrated embodiment is provided simultaneously with the magnetic band 7.

The device 1 can exhibit the credit card number/debit card number of the user, the relating period of validity, the name of the bank institute as well as the logo of the circuit to which it belong.

In case in which the user does not have a debit card and/or a credit card, the corresponding data may be absent, or the identification numbers of the credit card/debit card may be replaced by a plurality of zeros.

In prior art, instead, in order to carry paper documents, the latter were placed into folders or envelopes that nevertheless were not very practical, and in which the contents were liable to be damaged over time.

In addition, the device 1 is particularly advantageous for its practicalness under certain circumstances such as travels, medical examinations, stipulations of contracts of various type, etc.., when it is necessary having both paper documents, instruments for accessing to health services/bank services and for performing telematic payments, and identification documents and/or related to driving license (or driving licenses).

According to a further embodiment, not illustrated, the means of authorization/access to telematic payment services may be constituted by a NFC technology, that allows a contactless birdiectional communication between two or more components (suitable for exchanging data with said NFC technology)

According to an embodiment of the invention, the device 1 further comprises locating means (not showed in the attached figures), to allow the location of the same device.

In particular, the locating means may include, for example, a transponder aerial.

The locating means are useful because they allow traceability of the user, for example for security reasons.

In presence of a transponder aerial, the latter contains a one and only identification reference related to the user, which can be identified at a certain distance from proximity aerials related to this technology.

According to another embodiment, not illustrated, the device 1 also includes the digital signature (or electronic signature) of the user, that in prior art was necessary associated to a so-called "smart card". In this way, the user have not to carry a further card with him.

The device 1 may also include means for storing biometric characteristics of the user (not illustrated in the attached figures).

In particular, the means for storing biometric features are suitable for storing fingerprints and/or iris and/or facial prints of the user. Therefore, with appropriate reading tools, it is possible to identify the user in a certain way: this is important especially to prevent faking of personal documents, and then to ensure security and prevent cheats.

In addition to the photo, then, the aforesaid means for storing biometric characteristics of the user allow a correct identification rate of the user of 100%, whenever it is needed.

The device 1 may also include an identification number 10 of the same device 1 and therefore of the user himself.

The above has been described by way of non-limiting example, such that any constructional variations are considered to fall within the aim of the claims below.

## Claims

1. Multi-function device (1) associated to a user,
**characterized in that** it comprises at least two of the following features:
at least a part of data related to a driving license (2);
at least a part of data related to a health insurance card (3, 9);
at least a part of data related to an identity card (4);
means of authorization/access to telematic payment services (5);
means for storing data (8).

2. Multi-function device (1) according to claim 1, wherein the aforementioned means of authorization/access to telematic payment services (5) are linked to ATM circuits and/or credit cards circuits.

3. Multi-function device (1) according to claim 1 or 2, wherein the aforementioned means of authorization/access to telematic payment services (5) include a security chip (6) and/or a magnetic band (7) and/or a NFC technology.

4. Multi-function device (1) according to any one of the preceding claims, wherein the aforesaid means for storing data include a USB memory (8).

5. Multi-function device (1) according to any one of the preceding claim, further comprising locating means, to allow the location of the same device (1).

6. Multi-function device (1) according to the preceding claim, in which the location means include a transponder aerial.

7. Multi-function device (1) according to any one of the preceding claims, further comprising means for storing biometric characteristics of the user.

8. Multi-function device (1) according to any one of the preceding claims, wherein the means for storing biometric characteristics of the user are suitable for storing fingerprints and/or iris and/or facial prints of the user.

9. Multi-function device (1) according to any one of the preceding claims, in which said means for storing data (8) include a NFC device.

10. Multi-function device (1) according to any one of the preceding claims, also comprising the digital signature of the user.
